# EUROPEAN PATENT SPECIFICATION

(11) **EP 3 823 440 B1**
(45) Date of publication and mention of the grant of the patent: **20.04.2022**
(21) Application number: 18746867.3
(22) Date of filing: 19.07.2018
(51) Int. Cl.: A01K 11/00

(54) **SYSTEM FOR SAMPLING TISSUE**
SYSTEM ZUR ENTNAHME VON GEWEBE
SYSTÈME DE PRÉLÈVEMENT DE TISSU

(43) Date of publication of application: 26.05.2021
(73) Proprietor: Datamars SA, 6814 Lamone (CH)
(72) Inventor: IORI, Marco, 6807 Taverne (CH); BONGIORNO, Alessandro, 20010 Pregnana Milanese (MI) (IT); PACHOUD, Damien, 6963 Pregassona (CH)
(74) Representative: M. Zardi & Co S.A.
(86) International application number: PCT/EP2018/069656
(87) International publication number: WO 2020/015832

(56) References cited:
- WO-A1-2013/060690
- WO-A1-2014/199312
- DE-B3-102012 023 493
- US-A1- 2014 249 449

## Description

### Field of application:

The present invention relates to a system for sampling tissue from an ear of an animal and to tag the animal, at sampling. More particularly, the invention relates to a system of the type cited above, including a male component, a female component and a tissue sample container. The present invention also relates to a method to sample tissue from an ear of an animal.

### Prior art

Systems for sampling tissue from an ear of an animal are known. Document WO 2013/060690 A1 discloses such as system.

These systems including a male component, a female component and a tissue sample container coupled to the female component.

To sample the tissue, the female component is put in contact with a side of the ear and a tip of the male component, having a cutting edge and a collecting part, is pushed against the ear, from the other side thereof, to cut a sample of the ear and to enter in a hole of the female component, where the male component is finally locked. While the male component remains attached to the female component, tagging and identifying the animal, the tip enters the tissue sample container, and may be detached from the female component, together with the tissue sample container, for delivery to laboratory.

These systems are much appreciated because, in one single operation, the animal is tagged and the tissue sample is obtained. However, they also have some drawbacks, especially due to the fact that the tissue sample may be damaged or lost during the operation, for instance when calves or other animals having thin ear's skins are sampled. Lost of the sample or insufficient quantity of tissue sample may however occur with any kind of animal, with the known system.

Indeed, the sample may fall outside the collecting part after cut. This may occur immediately after the cut, since the sample moves outside a peripheral edge of the cutting edge arranged on top of the collecting part, therefore never entering the collecting part. In other cases, the sample actually enters the collecting part after the cut but subsequently exits therefrom, due to inappropriate movements of the tip along the path towards the tissue sample container.

Often, the operator is not aware of a failure in collecting the tissue sample. Indeed, also when the tissue is lost during sampling, the tissue sample container is sealed by the tip of the male component which, at least in part, hides the interior of the container. Therefore, the operator may believe that sampling have occurred properly although, actually, no tissue sample is stored inside the container.

All these problems affects the daily practise of operators responsible for tagging and sampling, to the extent that they must pay particular care and attention to avoid losing or damaging the sample. Also with all due care, it is however not easy (if not impossible) to visually follows the sample along the path from where it is initially cut from the animal's ear to the tissue sample container, especially at the interface between the female component and the tissue sample container.

In the worst cases, the result may be that the operator believes to have correctly accomplished sampling but, actually, no sample has been taken in the container or a damaged sample is hidden inside the tip, in the container.

Failures in collecting the tissue sample as described above happen with all types of known system for sampling tissue, including the ones having a female component not provided with a hole for the tip of the male component but with a continuous surface, adapted to support the animal's ear at sampling. In particular, such a female component may be adopted in an attempt to avoid breaking tissue of thin ears but it is completely useless to prevent the problem at the base of the present invention, to improve trust-ability of sampling, to avoid losing the sample tissue and to reduce checks of the operator.

The problem at the base of the present invention is that of providing a system for sampling tissue of an animal which is more reliable, easy to be used and that indicates the presence of the tissue inside the container, overcoming all the problems that presently affect the prior art system.

### Summary of the invention

The idea of solution at the base of the present invention is to provide a system for sampling a tissue from an animal's ear including means to guide the sample into a collecting part, to keep the sample inside the collecting part, preventing any lost and to indicate the presence of the tissue inside the container, once sampling is made.

More particularly, the idea of solution is to provide the means to guide the sample as a protrusion, preferably coupled to the tissue sample container, arranged in a hole of a female component and adapted to contact and guide the tissue of the animal from before its cut.

For instance, the protrusion contacts the tissue of the animal when the female component is put in contact with a side of the ear of the animal, projecting outside the hole of the female component. The tip of a male component, including a sample collecting part with a cutting edge, is adapted to receive, a portion of the (ear) tissue of the animal which, on the other side of the ear, is contacted by the protrusion, also before the cut of the sample. The sample collecting part is adapted to receive also the protrusion, together with the sample, when the sample is cut from the ear, keeping the sample safely inside the tip of the male component, all along its way to the tissue sample container.

Accordingly, the protrusion guides the tissue at any stage of the tagging and sampling process, allowing an appropriate shape change of the ear, also in young calves, meaning that any possible lost or damage of the tissue is avoided, forcing the tissue to immediately enter in the sample collecting part, beginning from before the cut, and then remaining reliably inside it also after the cut.

For instance, the protrusion may be in a shape of a pin and the sample collecting part may be in the shape of a cylinder with opening through which the pin enters, substantially surrounded by the tissue, this last remaining safely arranged between the external surface of the pin and the internal surface of the cylinder, from the beginning of the sampling operation.

Moreover, the protrusion is adapted to be broken by a passage of the tip inside the tissue sample container, and to follow the tissue sample and the sample collecting part, safely keeping the sample between the protrusion and the side of the sample collecting part, inside the tissue sample container, remaining in it as an evidence of a correct sampling. Accordingly, the operator may ascertain the presence of a sample in the tissue sample container by easily checking the presence of the protrusion inside it. At that stage, while the male component remains attached to the female component, as a tag of the animal, the tip is in the tissue sample container, and may be detached from the female component, together with the tissue sample container.

According to the solution idea given above, the technical problem is solved by a system for sampling tissue from an ear of an animal according to claim 1.

Advantageously, the protrusion is arranged in the hole to guide the tissue of the animal during the entire sampling process, from punching the ear to collecting the sample tissue in the tissue sample container, preventing the sample tissue to fall out from the sample collecting part, before closure of the tissue sample container. This is particularly advantageous when young animals are sampled since their skin is very thin and, if excessively bent, may fall apart from thesample collecting part. The protrusion is designed to remain into the tissue sample container as indicator of presence of the tissue sample inside the sample collecting part, after sampling.

According to the invention, the tissue sample container includes support means, arranged above the sealed membrane or cover of the tissue sample container for supporting the protrusion, the support means are adapted to be cut by the cutting edge of the maletip.

In one of the possible embodiments according to the present invention, a base of the sample collecting part includes a seat for an upper portion of the protrusion, the seat being adapted to keep the protrusion in the sample collecting part.

The protrusion is visible inside the tissue sample container for indicating presence of a tissue sample in the sample container. Preferably, the protrusion has a colour different from a colour of the tissue sample container and at least one of a peripheral edge, a bottom or a sealed membrane or cover of the tissue sample container is transparent.

According to the invention, the support means includes a ring having at least one radial portion, preferably four radial portions connected at the centre of the ring, supporting the protrusion at the middle of the ring.

The radial portion preferably includes at least one weakening line.

According to the present invention, the ring includes fastening means projecting towards the opening of the tissue sample container, and the detachable sample collecting part comprises an engagement profile adapted to be blocked by the fastening means.

Preferably, the upper portion of the protrusion is arranged at an opening of the hole through which the male component, more particularly the tip of the component including the sample collecting part, enters the hole.

For instance, the protrusion has a cylindrical shape and a diameter of the protrusion is between 1:6 and 1:2 of a diameter of the hole. Still as an example, the sample collecting part may have cylindrical shape.

The tissues sample container preferably includes a sample conserving agent and a cover or membrane closing the opening of the tissues sample container, wherein the cover or membrane is adapted to be broken by the cutting edge.

The tissues sample container may also include locking means to lock the female component before sampling, preferably an annular groove having shape coupling with an annular projection around the thorough hole. The locking means are releasable for detaching the counter plate from the tissue sample container, after sampling.

The tissues sample container, the female component and the male component preferably comprise a respective flag, including identification information.

The problem at the base of the present invention is also solved by a method according to claim 12 including the following phases:
- contacting a side of an ear to be sampled with a female component having a hole, the female component being attached to a removable tissue sample container, wherein a protrusion, preferably associated to the tissue sample container, protrudes from the hole, to contact the ear of the animal from said side of the ear;
- coupling a male component to the female component by positioning a sample collecting part having a cutting edge, on the opposite side of the ear of the animal, in a position where the protrusion may enter the sample container part, guiding the tissue of the animal into the sample collecting part beginning from before the cut;
- pushing the male component towards the female component to cut the tissue sample from the ear, wherein the protrusion keep forcing the sample into the sample collecting part during said step of pushing;
- entering the sample collecting part with the sampled tissue into the tissue sample container.

Further advantages and features of the system for sampling a tissue of an ear animal according to the present invention will be apparent from a embodiment given with reference to the annexed drawing only for exemplificative and not limitative purpose.

### Brief description of the drawings

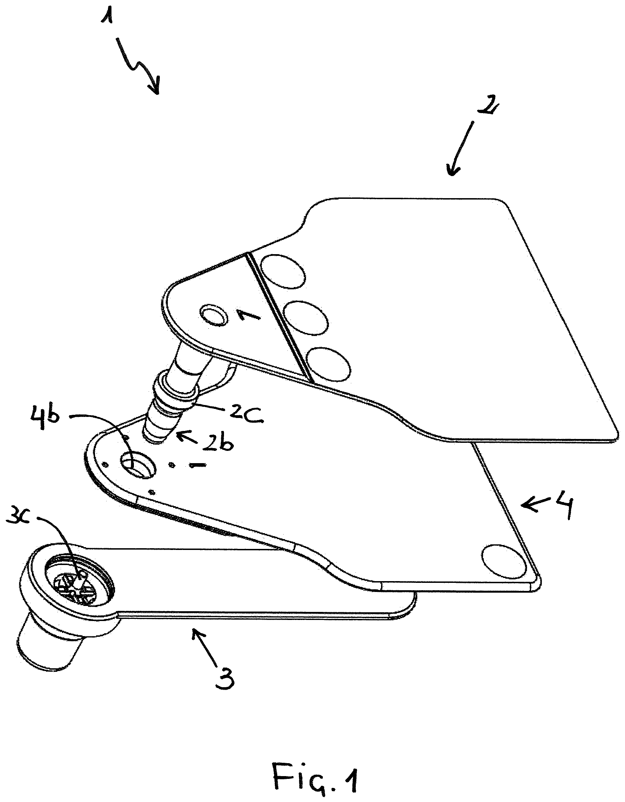

Figure 1 is a perspective view of the system for sampling a tissue of an animal according to the present invention.
Figure 2 is a perspective view of a tissue sample container of the system for sampling a tissue according to figure 1.

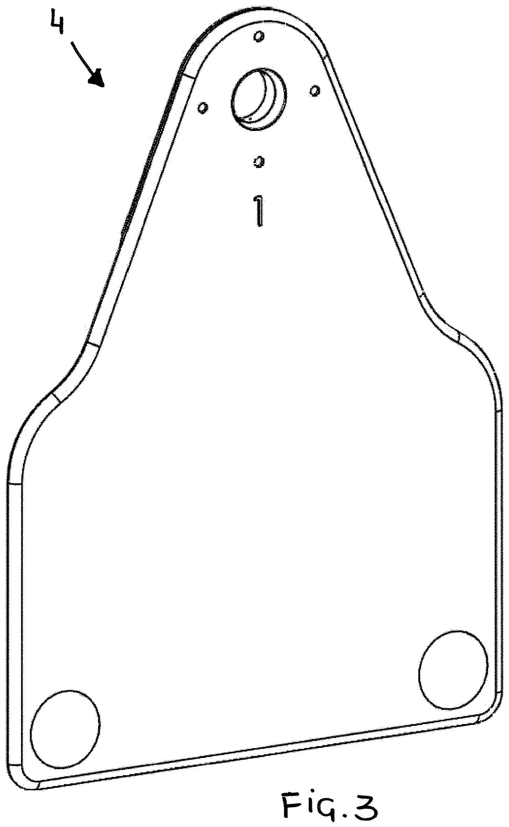

Figure 3 is a perspective view of a female component of the system for sampling a tissue according to figure 1.

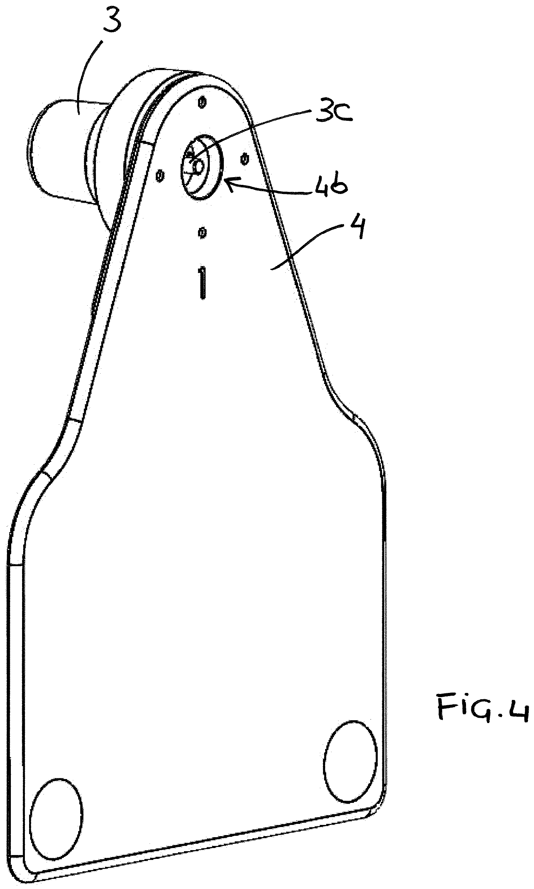

Figure 4 is a perspective view of the tissue sample container coupled to the female component of figures 2 and 3.
Figure 5 is a section view of a male component, the female component and the tissue sample container of the system for sampling a tissue according to figure 1, before coupling the male component with the female component.

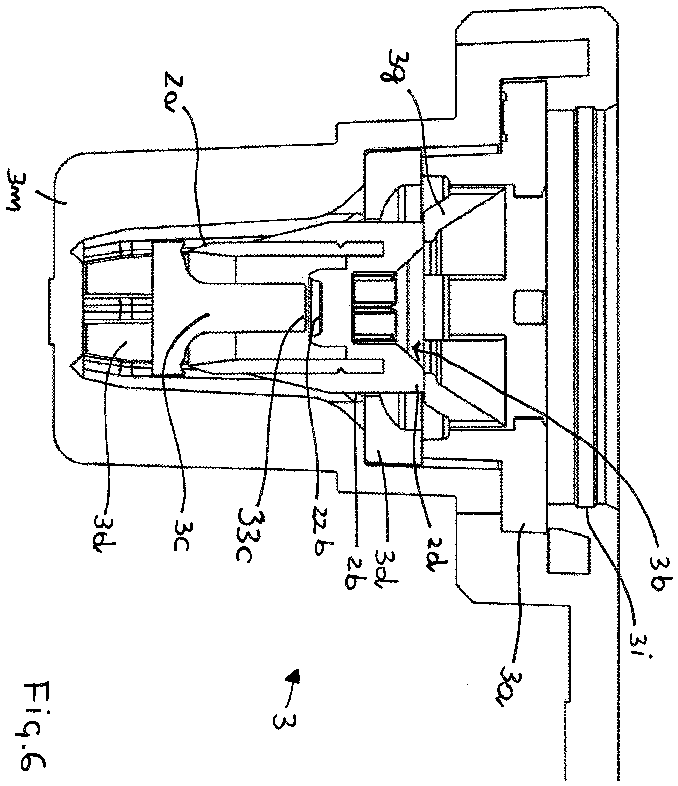

Figure 6 is a section view of the tissue sample container of the figure 2, after insertion of a sample collecting part 2b of the male component of figure 5 into the tissue sample container.

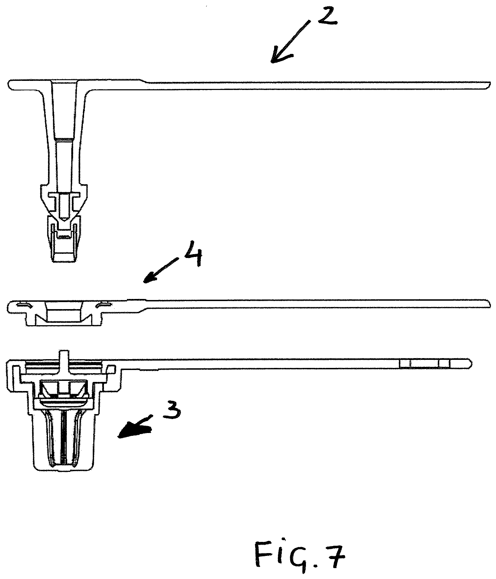

Figure 7 is a section view of the male component, the female component and the tissue sample container of the system for sampling a tissue according to figure 1, with detached parts.

### Detailed description

With reference to the annexed drawing, a system for sampling tissue from an animal's ear according to the present invention is schematically represented and indicated with reference number 1.

To sample the tissue from the animal's ear, a female component 4 is put in contact with a side of the ear and a male component 2, having a cutting edge 2a and a sample collecting part 2b, is pushed against the ear, from the other side thereof, to cut a sample of the ear and to enter in a hole 4b of the female component 4, where the male component 2 is finally locked, tagging the animal.

The sample collecting part 2b is adapted to enter a tissue sample container 3, attached to the female component 4 on the side thereof which is not in contact with the animal's ear, and may be detached from the female component 4, together with the tissue sample container. For instance, the tissues sample container 3 includes locking means 3i to lock the female component 4, preferably an annular groove 3i having shape coupling with an annular projection 4c around the thorough hole 4b; the locking means 3i are releasable for detaching the female component 4 from the tissue sample container 3. Of course, nothing prevents using a different removable coupling between the female component and the tissue sample container.

The male component 2 includes an engagement profile 2c, adapted to engage the female component and to remain attached thereto for tagging the animal. In this respect, the female component 4 includes retaining means 4a for retaining the engagement profile 2c of the male component 2, once the engagement profile 2c has passed the hole 4b.

The sample collecting part 2b is detachable from the male component 2 below the engagement profile 2c, so as to remain in the tissue sample container 3, after sampling, when the tissues sample container 3 is detached from the female component 4.

The tissue sample container 3 including an opening 3b arranged below the hole 4b of the female component 4b (when the female component 4 is attached to the tissue sample container 3), for receiving the sample collecting part 2b and the tissue sampled. The opening 3b is closed by a sealed membrane or a cover 3d. Preferably, the tissues sample container 3 includes a sample conserving agent and a cover or membrane 3h closing the opening 3b of the tissues sample container 3, wherein the cutting edge 2a are adapted to cut also the cover or membrane 3d.

Preferably, the tissues sample container 3, the female component 4 and the male component 2 comprise a respective flag, including visual identification information and or RFID means.

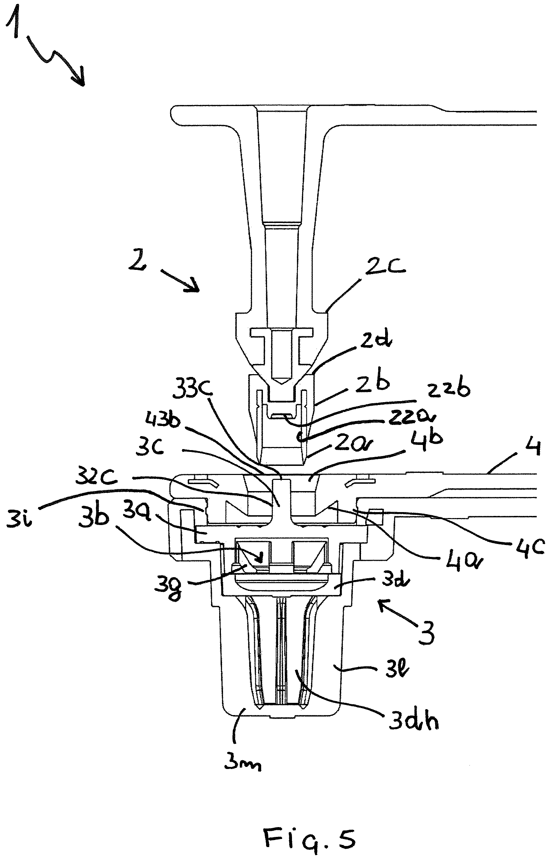

According to the present invention, the tissue sample container 3 includes a protrusion 3c, preferably a pin, arranged at least in part in the hole 4b of the female component 4 (when the female component 4 is attached to the tissue sample container 3), preferably protruding further than a surface of the female component 4 adapted to contact the animal's ear. Nothing prevents that the protrusion 3c does not protrude further than the surface of the female component 4, for instance remaining in the thickness of the female component 4, inside the hole 4b (fig. 5).

In the embodiment given with reference to the drawings, the protrusion 3c is at the centre of the hole 4b and it has the form of a cylindrical pin 3c. However, nothing prevents that, for instance, the pin has different cross section (triangular section, quadrangular section, ...) or that the pin is not at the centre of the hole 4b, for instance that more than one pin are arranged around the centre of the hole 4b.

The sample collecting part 2b is adapted to inserted in the hole 4b, on the protrusion 3c. In use, at the beginning of the sampling operation, the animal's ear is put in contact with a surface of the female 4. In the area of the hole 4b, the animal's ear is not contacted by the surface of the female 4, and it is arranged between the cutting edge 2a and the protrusion 3c.

When the male component 2 is pushed towards the female component 4 (using a tool for tagging, for instance having an arm attached to the female component and another arm to the male component), the portion of the animal's ear in the area of the hole 4b is driven inside the sample collecting part 2b by the protrusion 3c, in particular by an upper portion 33c thereof. At the initial stage of tagging, when the male component and the female component start approaching one to the other, the animal's ear may be not still cut. The ear is cut at a certain position along the way of the sample collecting part 2b towards the tissue sample container 3, depending of the features (thickness, flexibility, age, ...) of the animal's ear.

However, according to the present invention, at any position in which the animal's ear is cut, the corresponding tissue sample, is safely stocked inside the sample collecting part 2b, substantially between the protrusion 3c and the inner surface 22a of the sample collecting part 2b. Preferably, a space between the inner surface 22a of the sample collecting part 2b and the protrusion 3c is between 1 mm and 5 mm, the size being selected by design, depending of the features of the animal to be sampled. In one embodiment of the invention, the protrusion 3c has a cylindrical shape and a diameter of the protrusion 3c is between 1:6 and 1:2 of a diameter of the hole 4b.

According to the invention, the protrusion 3c is supported by support means 3a associated to the tissue sample container 3, for instance arranged above the sealed membrane or cover 3d of the tissue sample container 3. The cutting edge 2a of the male component 2 is adapted to cut also the support means 3a. Substantially, once the support means 3a are cut in the area of the cutting edge 2a, the protrusion 3c and a portion of the support means 3a remained attached to it after cut enter the sample collecting part 2b.

Preferably, a base 22b of the sample collecting part 2b, opposite to the opening where the cutting edge 2c is arranged, includes a seat for an upper portion 33c of the protrusion 3c. The seat keeps the protrusion 3c centred in the sample collecting part 2b, after sampling.

Also the sealed membrane 3d is adapted to be broken by the cutting edge 2a, when the sample collecting part 2b enters the housing 3dh of the tissue sample container 3. The protrusion 3c is visible in the housing 3dh, for indicating presence of the tissue sample in the sample container 3. In this respect, the protrusion 3c has a colour different from a colour of the tissue sample container 3 and at least one of a peripheral edge 31, a bottom 3m or the sealed membrane 3d of the tissue sample container 3 is transparent, to allow clear visibility of the protrusion. The operator may easily derive the presence of the sample and assume than an appropriate sampling has been executed by checking presence of the protrusion 3c in the hosing 3dh, being such a presence a sure sign of presence of the tissue sample. Just in absence of the protrusion, the sample may have been lost.

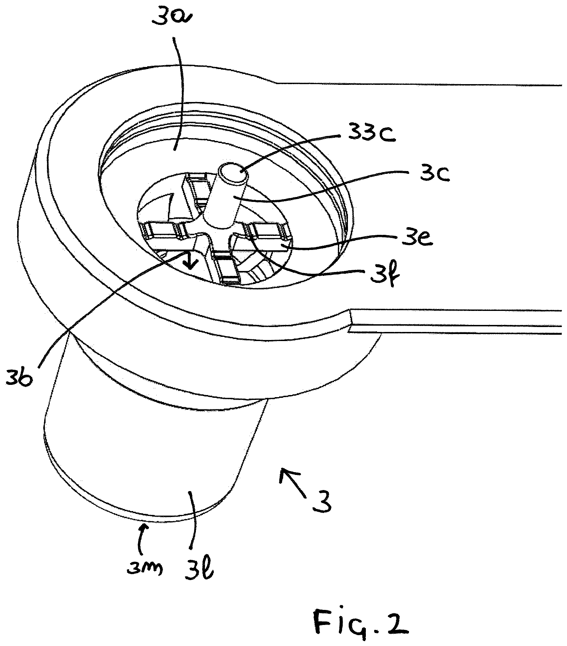

According to the invention, as depicted in figure 2, the support means 3a includes a ring 3a having at least one radial portion 3e, preferably four radial portions connected at the centre of the ring 3a, supporting the protrusion 3c at the middle of the ring 3a. The radial portion 3e includes at least one weakening line 3f.

The ring 3a is attached to the top of the housing 3dh by a plastic member, preferably thermo moulded over a peripheral flange of the ring 3a and the housing 3dh. The plastic member may form the flag for visual identification of the tissue sample container 3.

When the sample collecting part 2b enters the housing 3dh, it forms a plug for the housing 3dh, at the top of the tissues sample container 3, to protect the tissue sample inside it. In this respect, it is provided that the ring 3a includes fastening means 3g projecting towards the opening 3b of the tissue sample container 3 (i.e. towards the housing 3dh), and that the detachable sample collecting part 2b comprises an engagement profile 2d adapted to be blocked by the fastening means 3g.

Once the engagement profile 2d is blocked by the fastening means 3g, the tissues sample container may be pulled away from the female component, for detachment, this resulting in the end with the detachment of the sample collecting part 2b from the remaining portion of the male component from.

Several advantages derives from the structure and the use of the system for sampling according to the present invention. Especially, the system avoids loss of the sample at any stage of the procedure of sampling, since the ear of the animal in the area to be sampled is driven immediately by the protrusion in the sample collecting part, the protrusion force the sample derived from the cut to stay into the sample collecting part, and the protrusion move together with the sample collecting part trough the hole of the female, up to the housing of the container. In the housing, the protrusion is an indicator of a successful sampling and helps the operators to speed up controls and avoid repetition of samplings.

The system for sampling tissue of the present invention is applied to the animal's ear by means of an ear tag applicator of the type hereafter briefly disclosed. The applicator has two arms adapted to move between a closed position, where one arm is closed on the other arm, and an open position, when the arms are distanced apart. Two corresponding handles, preferably formed on the arms, extend beyond a point of rotation of the arms, and are closed, to close the applicator (the arms thereof), or opened to open it.

A first or upper arm includes a stem, preferably a metal stem, adapted to be coupled with the male component, preferably with the metal stem inside a cavity extending along an axis of the male component. A second or bottom arm includes a seat for the female component and the tissue sample container, the seat being arranged with respect to the first arm so that the hole of the female component, when placed in the seat, receives the tip of the male component, when placed in the first arm with the applicator closed.

## Claims

1. System (1) for sampling tissue from an ear of an animal including:
- a male component (2) including an engagement profile (2c) and a detachable sample collecting part (2b) with a cutting edge (2a);
- a female component (4) including a hole (4b) and retaining means (4a) for retaining the engagement profile (2c) of the male component (2), once the engagement profile (2c) has passed the hole (4b);
- a tissue sample container (3) including an opening (3b) arranged below the hole (4b) of the female component (4b), for receiving the sample collecting part (2b) and the tissue sampled, the opening (3b) being closed by a sealed membrane or a cover (3d);
- the tissue sample container (3) includes a protrusion (3c) arranged at least in part in the hole (4b) of the female component (4),
wherein the tissue sample container (3) includes support means (3a) arranged above the sealed membrane or cover (3d),
the support means (3a) include a ring (3a) disposed below the female component (4) when the female component (4) is attached to the tissue sample container (3), the ring (3a) having at least one radial portion (3e) supporting the protrusion (3c) at the middle of the ring (3a), wherein the ring (3a) is attached to a top of a housing (3dh) of the tissue sample container (3) by a plastic member thermo moulded over a peripheral flange of the ring (3a) and the housing (3dh),
the ring (3a) including fastening means (3g) projecting towards the opening (3b) of the tissue sample container (3), the detachable sample collecting part (2b) comprising an engagement profile (2d) adapted to be blocked by the fastening means (3g), and wherein the support means (3a) are adapted to be cut by the cutting edge (2a) of the male component (2).

2. System (1) according to claim 1 wherein a base (22b) of the sample collecting part (2b) includes a seat for an upper portion (33c) of the protrusion (3c), the seat being adapted to keep the protrusion (3c) in the sample collecting part (2b), after sampling, said protrusion (3c) being visible in the tissue sample container (3) for indicating presence of a tissue sample in the sample container (3).

3. System (1) according to claim 1 wherein the protrusion (3c) has a colour different from a colour of the tissue sample container (3) and wherein at least one of a peripheral edge (31), a bottom (3m) or sealed membrane or a cover (3d) of the tissue sample container (3) is transparent.

4. System (1) according to claim 1 wherein the ring (3a) has four radial portions connected at the centre of the ring (3a), supporting the protrusion (3c) at the middle of the ring (3a).

5. System (1) according to claim 1 wherein the radial portion (3e) includes at least one weakening line (3f).

6. System (1) according to claim 1 wherein an upper portion (33c) of the protrusion (3c) is arranged at an opening (43b) of the hole (4b) when the female component (4) is attached to the tissue sample container (3) .

7. System (1) according to claim 1 wherein the protrusion (3c) has a cylindrical shape and a diameter of the protrusion (3c) is between 1:6 and 1:2 of a diameter of the hole (4b).

8. System (1) according to claim 1 wherein the tissue sample container (3) includes a sample conserving agent.

9. System (1) according to claim 1 wherein the tissue sample container (3) includes locking means (3i) to lock the female component (4), preferably an annular groove (3i) having shape coupling with an annular projection (4c) around the female hole (4b), wherein the locking means (3i) are releasable for detaching the counter plate (4) from the tissue sample container (3).

10. System (1) according to claim 1 wherein the tissue sample container (3), the female component (4) and the male component (2) comprise a respective flag, including identification information.

11. System (1) according to claim 5 wherein said radial portion (3e) including the at least one weakening line (3f) is below the hole (4b).

12. Method for sampling a tissue from an ear of an animal using the system according to any one of claims 1-11, including:
- contacting a side of an ear to be sampled with a female component (4) having a hole (4b), the female component (4) being attached to a removable tissue sample container (3), wherein a protrusion (3c), associated to the tissue sample container, protrudes in or from the hole (4b), to contact the ear of the animal;
- coupling a male component (2) to the female component by positioning a sample collecting part (2b) having a cutting edge (2a), on the opposite side of the ear of the animal, in a position where the protrusion (3c) may enter the sample collecting part (2b), guiding the tissue of the animal into the sample collecting part (2b) from before the cut;
- pushing the male component (2) towards the female component (4) to cut the tissue sample from the ear, wherein the protrusion (3c) keep forcing the sample into the sample collecting part (2b) during said step of pushing;
- entering the sample collecting part (2b) with the sampled tissue into the tissue sample container (3),
wherein the tissue sample container (3) includes support means (3a), arranged above a sealed membrane or cover (3d) of the tissue sample container (3) and supporting the protrusion (3c), the support means (3a) being cut by the cutting edge (2a) of the male component (2), wherein
the support means (3a) include a ring (3a) disposed below the female component when the female component (4) is attached to the tissue sample container (3), said ring (3a) having at least one radial portion (3e) supporting the protrusion (3c) at the middle of the ring (3a), wherein the ring (3a) is attached to a top of a housing (3dh) of the tissue sample container (3) by a plastic member thermo moulded over a peripheral flange of the ring (3a) and the housing (3dh), wherein
the ring (3a) includes fastening means (3g) projecting towards the opening (3b) of the tissue sample container (3), and wherein the detachable sample collecting part (2b) comprises an engagement profile (2d) blocked by the fastening means (3g) when the sample collecting part enters the tissue sample container (3).

## Patentansprüche

1. System (1) zur Entnahme von Gewebeproben von einem Ohr eines Tieres, umfassend:
- eine männliche Komponente (2) mit einem Eingriffsprofil (2c) und einem abnehmbaren Probensammelteil (2b) mit einer Schneidkante (2a);
- eine weibliche Komponente (4) mit einem Loch (4b) und Haltemitteln (4a) zum Zurückhalten des Eingriffsprofils (2c) der männlichen Komponente (2), sobald das Eingriffsprofil (2c) das Loch (4b) passiert hat;
- einen Gewebeprobenbehälter (3) mit einer Öffnung (3b), die unterhalb des Lochs (4b) der weiblichen Komponente (4b) angeordnet ist, zur Aufnahme des Probensammelteils (2b) und des entnommenen Gewebes, wobei die Öffnung (3b) durch eine abgedichtete Membran oder eine Abdeckung (3d) verschlossen ist;
- wobei der Gewebeprobenbehälter (3) einen Vorsprung (3c) aufweist, der zumindest teilweise in dem Loch (4b) der weiblichen Komponente (4) angeordnet ist,
wobei der Gewebeprobenbehälter (3) Stützmittel (3a) enthält, die oberhalb der abgedichteten Membran oder Abdeckung (3d) angeordnet sind,
wobei die Stützmittel (3a) einen Ring (3a) umfassen, der unterhalb der weiblichen Komponente (4) angeordnet ist, wenn die weibliche Komponente (4) an dem Gewebeprobenbehälter (3) angebracht ist, wobei der Ring (3a) mindestens einen radialen Abschnitt (3e) aufweist, der den Vorsprung (3c) in der Mitte des Rings (3a) stützt, wobei der Ring (3a) an einer Oberseite eines Gehäuses (3dh) des Gewebeprobenbehälters (3) durch ein Kunststoffelement angebracht ist, das über einen Umfangsflansch des Rings (3a) und das Gehäuse (3dh) thermogeformt ist,
wobei der Ring (3a) Befestigungsmittel (3g) aufweist, die in Richtung der Öffnung (3b) des Gewebeprobenbehälters (3) vorstehen, wobei das abnehmbare Probensammelteil (2b) ein Eingriffsprofil (2d) aufweist, das so angepasst ist, dass es durch die Befestigungsmittel (3g) blockiert wird, und wobei die Stützmittel (3a) so angepasst sind, dass sie durch die Schneidkante (2a) der männlichen Komponente (2) geschnitten werden.

2. System (1) nach Anspruch 1, wobei eine Basis (22b) des Probensammelteils (2b) einen Sitz für einen oberen Abschnitt (33c) des Vorsprungs (3c) aufweist, wobei der Sitz angepasst ist, um den Vorsprung (3c) nach der Probenahme in dem Probensammelteil (2b) zu halten, wobei der Vorsprung (3c) in dem Gewebeprobenbehälter (3) sichtbar ist, um das Vorhandensein einer Gewebeprobe in dem Probenbehälter (3) anzuzeigen.

3. System (1) nach Anspruch 1, wobei der Vorsprung (3c) eine Farbe hat, die sich von einer Farbe des Gewebeprobenbehälters (3) unterscheidet, und wobei mindestens einer von einem Umfangsrand (31), einem Boden (3m) oder einer abgedichteten Membran oder einer Abdeckung (3d) des Gewebeprobenbehälters (3) transparent ist.

4. System (1) nach Anspruch 1, wobei der Ring (3a) vier radiale Abschnitte aufweist, die in der Mitte des Rings (3a) verbunden sind und den Vorsprung (3c) in der Mitte des Rings (3a) tragen.

5. System (1) nach Anspruch 1, wobei der radiale Abschnitt (3e) mindestens eine Schwächungslinie (31) aufweist.

6. System (1) nach Anspruch 1, wobei ein oberer Teil (33c) des Vorsprungs (3c) an einer Öffnung (43b) des Lochs (4b) angeordnet ist, wenn die weibliche Komponente (4) an dem Gewebeprobenbehälter (3) angebracht ist.

7. System (1) nach Anspruch 1, wobei der Vorsprung (3c) eine zylindrische Form hat und ein Durchmesser des Vorsprungs (3c) zwischen 1:6 und 1:2 eines Durchmessers des Lochs (4b) beträgt.

8. System (1) nach Anspruch 1, wobei der Gewebeprobenbehälter (3) ein Probenkonservierungsmittel enthält.

9. System (1) nach Anspruch 1, wobei der Gewebeprobenbehälter (3) Verriegelungsmittel (3i) zum Verriegeln des weiblichen Teils (4), vorzugsweise eine ringförmige Nut (3i), die eine Formkopplung mit einem ringförmigen Vorsprung (4c) um das weibliche Loch (4b) herum aufweist, umfasst, wobei die Verriegelungsmittel (3i) lösbar sind, um die Gegenplatte (4) von dem Gewebeprobenbehälter (3) zu lösen.

10. System (1) nach Anspruch 1, wobei der Gewebeprobenbehälter (3), die weibliche Komponente (4) und die männliche Komponente (2) eine jeweilige Kennzeichnung aufweisen, die Identifikationsinformationen enthält.

11. System (1) nach Anspruch 5, wobei der radiale Abschnitt (3e) mit der mindestens einen Schwächungslinie (31) unterhalb des Lochs (4b) liegt.

12. Verfahren zur Entnahme einer Gewebeprobe von einem Ohr eines Tieres unter Verwendung des Systems nach einem der Ansprüche 1 bis 11, umfassend:
- Berühren einer Seite eines zu beprobenden Ohres mit einer weiblichen Komponente (4), die ein Loch (4b) aufweist, wobei die weibliche Komponente (4) an einem abnehmbaren Gewebeprobenbehälter (3) angebracht ist, wobei ein Vorsprung (3c), der dem Gewebeprobenbehälter zugeordnet ist, in das oder aus dem Loch (4b) ragt, um das Ohr des Tieres zu berühren;
- Koppeln einer männlichen Komponente (2) mit der weiblichen Komponente (4) durch Positionieren eines Probensammelteils (2b) mit einer Schneidkante (2a) auf der gegenüberliegenden Seite des Ohrs des Tiers in einer Position, in der der Vorsprung (3c) in das Probensammelteil (2b) eintreten kann und das Gewebe des Tiers in das Probensammelteil (2b) von vor dem Schnitt geführt wird;
- Schieben der männlichen Komponente (2) in Richtung der weiblichen Komponente (4), um die Gewebeprobe von dem Ohr zu schneiden, wobei der Vorsprung (3c) die Probe während des Schiebeschrittes weiterhin in den Probensammelteil (2b) drückt;
- Einführen des Probensammelteils (2b) mit dem entnommenen Gewebe in den Gewebeprobenbehälter (3),
wobei der Gewebeprobenbehälter (3) Stützmittel (3a) enthält, die über einer abgedichteten Membran oder Abdeckung (3d) des Gewebeprobenbehälters (3) angeordnet sind und den Vorsprung (3c) stützen, wobei die Stützmittel (3a) von der Schneidkante (2a) der männlichen Komponente (2) geschnitten werden, wobei die Stützmittel (3a) einen Ring (3a) umfassen, der unterhalb der weiblichen Komponente angeordnet ist, wenn die weibliche Komponente (4) an dem Gewebeprobenbehälter (3) angebracht ist, wobei der Ring (3a) mindestens einen radialen Abschnitt (3e) aufweist, der den Vorsprung (3c) in der Mitte des Rings (3a) stützt, wobei der Ring (3a) an einer Oberseite eines Gehäuses (3dh) des Gewebeprobenbehälters (3) durch ein Kunststoffelement befestigt ist, das über einen Umfangsflansch des Rings (3a) und das Gehäuse (3dh) thermogeformt ist, wobei der Ring (3a) Befestigungsmittel (3g) aufweist, die in Richtung der Öffnung (3b) des Gewebeprobenbehälters (3) vorstehen, und wobei der abnehmbare Probensammelteil (2b) ein Eingriffsprofil (2d) aufweist, das durch die Befestigungsmittel (3g) blockiert wird, wenn der Probensammelteil in den Gewebeprobenbehälter (3) eintritt.

## Revendications

1. Système (1) pour échantillonner un tissu à partir d'une oreille d'un animal, incluant :
- un composant mâle (2) qui inclut un profilé d'engagement (2c) et une partie de collecte d'échantillon détachable (2b) qui est munie d'un bord de coupe (2a) ;
- un composant femelle (4) qui inclut un trou (4b) et un moyen de retenue (4a) pour retenir le profilé d'engagement (2c) du composant mâle (2), une fois que le profilé d'engagement (2c) est passé au travers du trou (4b) ;
- un moyen de contenance d'échantillon de tissu (3) qui inclut une ouverture (3b) qui est agencée au-dessous du trou (4b) du composant femelle (4), pour recevoir la partie de collecte d'échantillon (2b) et le tissu qui a été échantillonné, l'ouverture (3b) étant fermée par une membrane étanche ou par un moyen de recouvrement (3d) ;
- le moyen de contenance d'échantillon de tissu (3) inclut une protubérance (3c) qui est agencée au moins en partie à l'intérieur du trou (4b) du composant femelle (4) ;
dans lequel le moyen de contenance d'échantillon de tissu (3) inclut un moyen de support (3a) qui est agencé au-dessus de la membrane étanche ou du moyen de recouvrement (3d) ;
le moyen de support (3a) inclut une bague (3a) qui est disposée au-dessous du composant femelle (4) lorsque le composant femelle (4) est lié au moyen de contenance d'échantillon de tissu (3), la bague (3a) comportant au moins une partie radiale (3e) qui supporte la protubérance (3c) au niveau du milieu de la bague (3a), dans lequel la bague (3a) est liée à un sommet d'un logement (3dh) du moyen de contenance d'échantillon de tissu (3) au moyen d'un élément en matière plastique qui est thermo-moulé au-dessus d'une bride périphérique de la bague (3a) et du logement (3dh) ;
la bague (3a) incluant un moyen de fixation (3g) qui fait saillie en direction de l'ouverture (3b) du moyen de contenance d'échantillon de tissu (3), la partie de collecte d'échantillon détachable (2b) comprenant un profilé d'engagement (2d) qui est adapté pour être bloqué par le moyen de fixation (3g) ; et dans lequel
le moyen de support (3a) est adapté pour être coupé par le bord de coupe (2a) du composant mâle (2).

2. Système (1) selon la revendication 1, dans lequel une base (22b) de la partie de collecte d'échantillon (2b) inclut un siège pour une partie supérieure (33c) de la protubérance (3c), le siège étant adapté pour maintenir la protubérance (3c) dans la partie de collecte d'échantillon (2b), après échantillonnage, ladite protubérance (3c) étant visible dans le moyen de contenance d'échantillon de tissu (3) pour indiquer la présence d'un échantillon de tissu dans le moyen de contenance d'échantillon de tissu (3).

3. Système (1) selon la revendication 1, dans lequel la protubérance (3c) présente une couleur qui est différente d'une couleur du moyen de contenance d'échantillon de tissu (3) et dans lequel au moins un élément structurel pris parmi un bord périphérique (3l), un fond (3m) et une membrane étanche ou un moyen de recouvrement (3d) du moyen de contenance d'échantillon de tissu (3) est transparent.

4. Système (1) selon la revendication 1, dans lequel la bague (3a) comporte quatre parties radiales qui sont connectées au niveau du centre de la bague (3a), lesquelles parties radiales supportent la protubérance (3c) au niveau du milieu de la bague (3a).

5. Système (1) selon la revendication 1, dans lequel la partie radiale (3e) inclut au moins une ligne de rupture (3f).

6. Système (1) selon la revendication 1, dans lequel une partie supérieure (33c) de la protubérance (3c) est agencée au niveau d'une ouverture (43b) du trou (4b) lorsque le composant femelle (4) est lié au moyen de contenance d'échantillon de tissu (3).

7. Système (1) selon la revendication 1, dans lequel la protubérance (3c) présente une forme cylindrique et un diamètre de la protubérance (3c) se situe entre 1:6 et 1:2 par rapport à un diamètre du trou (4b).

8. Système (1) selon la revendication 1, dans lequel le moyen de contenance d'échantillon de tissu (3) inclut un agent de conservation d'échantillon.

9. Système (1) selon la revendication 1, dans lequel le moyen de contenance d'échantillon de tissu (3) inclut un moyen de blocage (3i) pour bloquer le composant femelle (4), de préférence une gorge annulaire (3i) qui présente une forme en couplage avec une projection annulaire (4c) autour du trou femelle (4b), dans lequel le moyen de blocage (3i) peut être libéré pour détacher la contre-plaque/le composant femelle (4) vis-à-vis du moyen de contenance d'échantillon de tissu (3).

10. Système (1) selon la revendication 1, dans lequel le moyen de contenance d'échantillon de tissu (3), le composant femelle (4) et le composant mâle (2) comprennent un indicateur respectif, lequel indicateur inclut une information d'identification.

11. Système (1) selon la revendication 5, dans lequel ladite partie radiale (3e) qui inclut l'au moins une ligne de rupture (3f) est au-dessous du trou (4b).

12. Procédé pour échantillonner un tissu à partir d'une oreille d'un animal en utilisant le système selon l'une quelconque des revendications 1 à 11, incluant :
- la mise en contact d'un côté d'une oreille qui doit être échantillonnée avec un composant femelle (4) qui comporte un trou (4b), le composant femelle (4) étant lié à un moyen de contenance d'échantillon de tissu amovible (3), dans lequel une protubérance (3c), qui est associée au moyen de contenance d'échantillon de tissu, fait saillie à l'intérieur du trou ou depuis le trou (4b), pour une mise en contact avec l'oreille de l'animal ;
- le couplage d'un composant mâle (2) avec le composant femelle (4) en positionnant une partie de collecte d'échantillon (2b) qui comporte un bord de coupe (2a) sur le côté opposé de l'oreille de l'animal, dans une position dans laquelle la protubérance (3c) peut pénétrer à l'intérieur de la partie de collecte d'échantillon (2b), en guidant le tissu de l'animal à l'intérieur de la partie de collecte d'échantillon (2b) avant de pratiquer la coupe ;
- la poussée du composant mâle (2) en direction du composant femelle (4) pour couper l'échantillon de tissu à partir de l'oreille, dans lequel la protubérance (3c) continue à forcer l'échantillon à l'intérieur de la partie de collecte d'échantillon (2b) pendant ladite étape de poussée ;
- la pénétration de la partie de collecte d'échantillon (2b) qui est munie du tissu échantillonné à l'intérieur du moyen de contenance d'échantillon de tissu (3) ;
dans lequel le moyen de contenance d'échantillon de tissu (3) inclut un moyen de support (3a) qui est agencé au-dessus d'une membrane étanche ou d'un moyen de recouvrement (3d) du moyen de contenance d'échantillon de tissu (3) et qui supporte la protubérance (3c), le moyen de support (3a) étant coupé par le bord de coupe (2a) du composant mâle (2) ; dans lequel
le moyen de support (3a) inclut une bague (3a) qui est disposée au-dessous du composant femelle lorsque le composant femelle (4) est lié au moyen de contenance d'échantillon de tissu (3), la bague (3a) comportant au moins une partie radiale (3e) qui supporte la protubérance (3c) au niveau du milieu de la bague (3a), dans lequel la bague (3a) est liée à un sommet d'un logement (3dh) du moyen de contenance d'échantillon de tissu (3) au moyen d'un élément en matière plastique qui est thermo-moulé au-dessus d'une bride périphérique de la bague (3a) et du logement (3dh) ; dans lequel
la bague (3a) inclut un moyen de fixation (3g) qui fait saillie en direction de l'ouverture (3b) du moyen de contenance d'échantillon de tissu (3), et dans lequel la partie de collecte d'échantillon détachable (2b) comprend un profilé d'engagement (2d) qui est bloqué par le moyen de fixation (3g) lorsque la partie de collecte d'échantillon pénètre à l'intérieur du moyen de contenance d'échantillon de tissu (3).
